# EUROPEAN PATENT APPLICATION

(11) **EP 4 621 397 A1**
(43) Date of publication of application: **24.09.2025**
(21) Application number: 23891673.8
(22) Date of filing: 17.11.2023
(51) Int. Cl.: G01N 24/00, C12M 1/00, C12M 1/34, G01N 24/08, G01N 33/483, G01R 33/26, G01R 33/465

(54) **INTRACELLULAR QUANTUM METROLOGY DEVICE, INTRACELLULAR QUANTUM METROLOGY METHOD, AND CELL RETENTION DEVICE**

(30) Priority: 18.11.2022 JP 2022185254
(71) Applicant: National Institutes for Quantum Science and Technology, Chiba 263-8555 (JP)
(72) Inventor: ISHIWATA, Hitoshi, Chiba-shi, Chiba 263-8555 (JP); IGARASHI, Ryuji, Chiba-shi, Chiba 263-8555 (JP); KAMINAGA, Kiichi, Chiba-shi, Chiba 263-8555 (JP)
(74) Representative: Graf von Stosch, Andreas
(86) International application number: PCT/JP2023/041402
(87) International publication number: WO 2024/106528

(57) **Abstract**

Achieved is quantum measurement that can measure an intracellular state and that overcomes disadvantages of quantum measurement that uses nanoparticles. An intracellular quantum measurement device (1) includes a substrate (10) that has a surface on which a nanopillar (11) to be inserted into a cell (C) is formed, the nanopillar (11) having a tip part in which a quantum sensor is included.

## Description

### Technical Field

The present invention relates to an intracellular quantum measurement device and an intracellular quantum measurement method for measuring an intracellular state using a quantum sensor. Moreover, the present invention relates to a cell retention device which includes such an intracellular quantum measurement device.

### Background Art

In a case where a nitrogen atom exists in diamond crystal, a vacancy may be formed next to the nitrogen atom. At this time, a nitrogen-vacancy (NV) center which is the center between the nitrogen atom and the vacancy has (i) property that a quantum state changes according to a surrounding environment such as an electric field, a magnetic field, and a temperature and (ii) property that the quantum state can be read by fluorescence measurement. Therefore, the NV center can be used as a quantum sensor for detecting a surrounding environment. As a fluorescence microscope for carrying out fluorescence measurement, for example, fluorescence microscopes disclosed in Patent Literatures 1 and 2 and the like are known.

Such quantum sensors can be utilized to measure a state of a cell. For example, a nanoparticle of diamond including an NV center can be introduced into a cell to measure a state in the cell. Such a nanoparticle is expected to be utilized as a "next generation biomarker". In addition, a nano thin film of diamond including an NV center can be adhered to a cell membrane to measure a state of the cell membrane. Such a nano thin film is expected to be utilized as a "next generation cover slip".

### Citation List

### [Patent Literature]

[Patent Literature 1]
   Japanese Patent No. 5476206
[Patent Literature 2]
   Japanese Patent No. 6117812

### Summary of Invention

### Technical Problem

However, quantum measurement using a nanoparticle (next generation biomarker) has an advantage of being able to measure an intracellular state, whereas has disadvantages that (1) it is difficult to strictly set a measurement environment, (2) light emission intensity is low, (3) spinning property is poor, and (4) characteristics of respective quantum sensors (NV centers) are not uniform. A cause of these disadvantages is mainly that it is impossible to fix a position and an orientation of the quantum sensor. As a result, for example, it becomes difficult to precisely adjust a direction of a magnetic field which is applied to a quantum sensor and to optimally select a pulse shape of excitation light with which the quantum sensor is to be irradiated, and the like. Therefore, as a method of quantum measurement, DC measurement (measurement to capture a change in light emission intensity using a light emission state) is used.

Meanwhile, quantum measurement using a diamond thin film (next generation cover slip) has advantages that (1) it is easy to strictly set a measurement environment, (2) light emission intensity is high, (3) spinning property is good, and (4) characteristics of respective quantum sensors (NV centers) are uniform, whereas has a disadvantage that it is impossible to measure an intracellular state. A cause of these advantages is mainly that it is possible to fix a position and an orientation of the quantum sensor. As a result, for example, it becomes easy to precisely adjust a direction of a magnetic field which is applied to a quantum sensor and to optimally select a pulse shape of excitation light with which the quantum sensor is to be irradiated, and the like. Therefore, as a method of quantum measurement, AC measurement (measurement to capture a change in phase of a quantum level using a superposition state) is used.

An aspect of the present invention is accomplished in view of the above problem, and an object thereof is to achieve quantum measurement that can measure an intracellular state and that overcomes disadvantages of quantum measurement that uses nanoparticles.

### Solution to Problem

In order to attain the object, an intracellular quantum measurement device in accordance with an aspect of the present invention includes: a substrate that has a surface on which a nanopillar is formed, the nanopillar being to be inserted into a cell, and the nanopillar having a tip part in which a quantum sensor is included.

In order to attain the object, in an intracellular quantum measurement method in accordance with an aspect of the present invention, a substrate that has a surface on which a nanopillar is formed is used to carry out quantum measurement in a state in which the nanopillar is inserted into a cell, the nanopillar having a tip part in which a quantum sensor is included.

### Advantageous Effects of Invention

According to an aspect of the present invention, it is possible to achieve quantum measurement that can measure an intracellular state and that overcomes disadvantages of quantum measurement that uses nanoparticles.

### Brief Description of Drawings

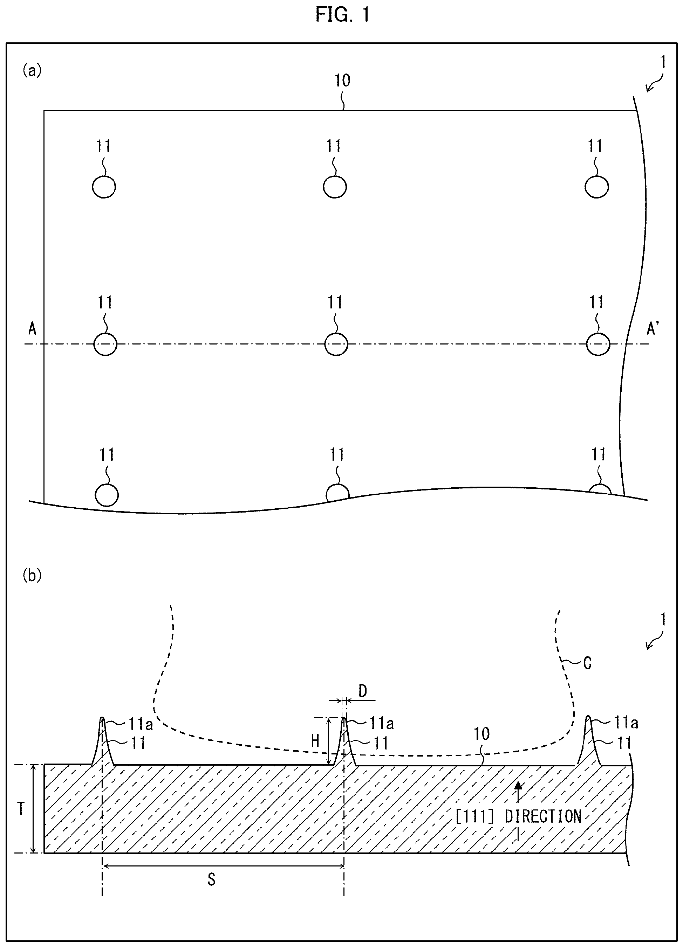

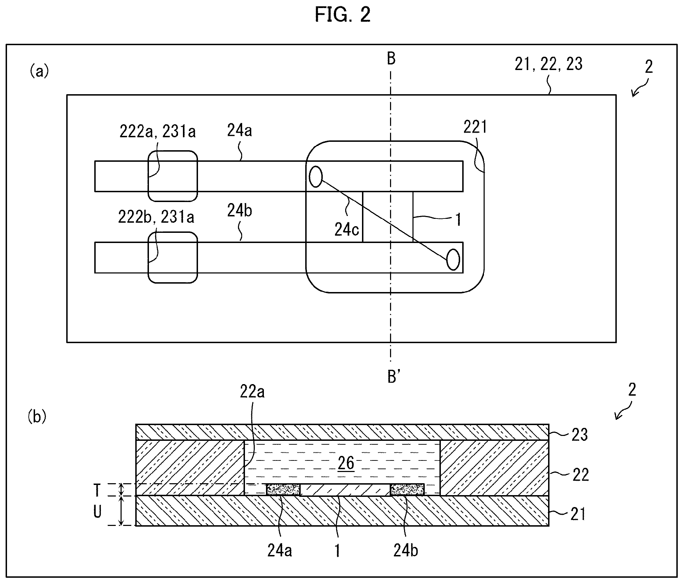

(a) of Fig. 1 is a plan view illustrating an intracellular quantum measurement device in accordance with an embodiment of the present invention, and (b) of Fig. 1 is a cross-sectional view illustrating the intracellular quantum measurement device.
(a) of Fig. 2 is a plan view illustrating a cell retention device which includes the intracellular quantum measurement device illustrated in Fig. 1, and (b) of Fig. 2 is a cross-sectional view illustrating the cell retention device.
Fig. 3 is a configuration diagram illustrating an intracellular quantum measurement system which includes the cell retention device illustrated in Fig. 2.
Fig. 4 is a diagram illustrating a working example of the intracellular quantum measurement system illustrated in Fig. 3. (a) of Fig. 4 shows an image obtained by imaging a surface of the intracellular quantum measurement device. (b) of Fig. 4 shows an image obtained by imaging a nanopillar of the intracellular quantum measurement device. (c) and (d) of Fig. 4 each show an image obtained by imaging a Hepa 1-6 cell which is cultured by the cell retention device.
Fig. 5 is a graph illustrating a working example of the intracellular quantum measurement system illustrated in Fig. 3, and represents τ dependency of normalized contrast obtained by Rabi measurement.
Fig. 6 is a graph illustrating a working example of the intracellular quantum measurement system illustrated in Fig. 3, and represents τ dependency of normalized contrast obtained by NMR measurement.
Fig. 7 is a graph illustrating a working example of the intracellular quantum measurement system illustrated in Fig. 3, and represents time dependency of normalized contrast.
(a) of Fig. 8 is a diagram illustrating a nanopillar, and (b) of Fig. 8 is an expanded schematic diagram illustrating a quantum sensor (NV center) which is disposed in a tip part of the nanopillar.
Fig. 9 is a diagram illustrating a pulse sequence for carrying out quantum manipulation on a quantum sensor and a radical or the like in the vicinity of the quantum sensor in the nanopillar illustrated in Fig. 8.
Fig. 10 is a graph illustrating a working example of a result obtained using the pulse sequence illustrated in Fig. 9, and represents tau dependency of contrast obtained by ESR measurement using double quantum manipulation.

### Description of Embodiments

### [Intracellular quantum measurement device]

The following description will discuss an intracellular quantum measurement device 1 in accordance with an embodiment of the present invention, with reference to Fig. 1. (a) of Fig. 1 is a plan view illustrating the intracellular quantum measurement device 1, and (b) of Fig. 1 is a cross-sectional view illustrating the intracellular quantum measurement device 1. (b) of Fig. 1 illustrates the A-A' cross section (see (a) of Fig. 1) of the intracellular quantum measurement device 1.

The intracellular quantum measurement device 1 includes a substrate 10 having a surface on which a plurality of nanopillars 11 are formed. The nanopillars 11 are each a structure which is to be inserted into a cell C and has a columnar, conical, or pyramidal shape (in the present embodiment, a structure having a root part in a conical shape and a tip part in a columnar shape). A tip part 11a of each of the nanopillars 11 includes a quantum sensor. The nanopillars 11 are disposed on the surface of the substrate 10 at regular intervals (in the present embodiment, at lattice points of a square lattice).

In the present embodiment, the substrate 10 and the nanopillars 11 are constituted by diamond crystal. In the present embodiment, the tip part 11a of each of the nanopillars 11 includes an NV center which is constituted by one nitrogen atom and one vacancy that is adjacent to the nitrogen atom. The NV center is used as a quantum sensor. Note that, in order to accurately adjust a magnetic field to be applied to the quantum sensor, a <111> direction of the diamond crystal is made to conform to the normal direction to the surface of the substrate 11.

A height H of the nanopillar 11 and a diameter D of the tip part 11a are determined so that intracellular measurement can be achieved (e.g., penetration through a cell membrane can be achieved, adhesion of cells including a floating cell can be achieved) while taking into consideration a physical condition which is to sufficiently enhance fluorescence extraction efficiency and a biological condition which is to sufficiently reduce toxicity with respect to cells.

From the above point of view, the height H of the nanopillar 11 may be 1 µm or more and 20 µm or less, is preferably 1 µm or more and 5 µm or less, more preferably 2 µm or more and 4 µm or less, further preferably 2.5 µm or more and 3.5 µm or less. In the present embodiment, the height H of the nanopillar 11 is 3.2 µm.

From the above point of view, the diameter D of the tip part 11a of the nanopillar 11 may be 100 nm or more and 1000 nm or less, is preferably 100 nm or more and 500 nm or less, more preferably 200 nm or more and 400 nm or less, further preferably 250 nm or more and 350 nm or less. In the present embodiment, the diameter D of the tip part 11a of the nanopillar 11 is 300 nm.

From the viewpoint of sufficiently increasing fluorescence extraction efficiency, the thickness T of the substrate 10 is preferably 50 µm or less. In the present embodiment, the thickness T of the substrate 10 is 40 µm.

Further, an interval (center-to-center distance) D between adjacent nanopillars 11 is determined to be approximately 1/2 of a size of a cell to be measured in order to increase a probability that any of the nanopillars 11 is inserted into a cell which is cultured in the cell retention device 2 (described later).

From the above point of view, the interval D between adjacent nanopillars 11 is preferably 10 µm or more and 60 µm or less. For example, in a case where a subject to be measured is a somatic cell (approximately 20 µm to 50 µm) of a mammal, it is more preferable that the interval D between adjacent nanopillars 11 is 10 µm or more and 25 µm or less. In a case where a subject to be measured is a reproductive cell (approximately 50 µm to 80 µm) of a mammal, it is more preferable that the interval D between adjacent nanopillars 11 is 25 µm or more and 40 µm or less. In a case where a subject to be measured is a cell (approximately 80 µm to 120 µm) of a plant, it is more preferable that the interval D between adjacent nanopillars 11 is 40 µm or more and 60 µm or less.

According to the intracellular quantum measurement device 1 in accordance with the present embodiment, it is possible to carry out quantum measurement in a state in which the tip part 11a of the nanopillar 11 is inserted into the cell C. Here, quantum measurement refers to identifying a quantum state of a quantum sensor by measuring fluorescence emitted from the quantum sensor and inferring a state around the quantum sensor from the identified quantum state. In the present embodiment, inferring a state around the quantum sensor is, in other words, inferring a state inside the cell C.

Note that the nanopillar 11 is not isolated and floating in a cell unlike a nanoparticle, but is formed on the surface of the substrate like a nano thin film. Therefore, AC measurement can be carried out as with quantum measurement using a diamond thin film, and it is therefore possible to precisely measure a state inside the cell C using a high-sensitivity spin analysis technique such as NMR and ESR. That is, while overcoming disadvantages of quantum measurement that uses nanoparticles, it is possible to measure a state inside the cell C.

In the present embodiment, the configuration is employed in which a plurality of nanopillars 11 are formed on the surface of the substrate 10. Note, however, that the present invention is not limited to this configuration. That is, a configuration may be employed in which a single nanopillar 11 is formed on the surface of the substrate 10. Note, however, that, by employing the configuration in which a plurality of nanopillars 11 are formed on the surface of the substrate 10 as in the present embodiment, a further effect can be brought about which is to make it possible to simultaneously carry out quantum measurement of a plurality of cells (e.g., a plurality of cells constituting a living tissue). Such simultaneous measurement is carried out in a state in which tip parts of the plurality of nanopillars 11 are respectively inserted into the plurality of cells.

### [Cell retention device]

The following description will discuss a cell retention device 2 including the foregoing intracellular quantum measurement device 1, with reference to Fig. 2. (a) of Fig. 2 is a plan view illustrating the cell retention device 2, and (b) of Fig. 2 is a cross-sectional view illustrating the cell retention device 2. (b) of Fig. 2 illustrates the B-B' cross section (see (a) of Fig. 2) of the cell retention device 2.

The cell retention device 2 includes a first substrate 21, a second substrate 22, and a third substrate 23. The first substrate 21 functions as a cover slip. The first substrate 21 may be made of an arbitrary material. Examples of the material include an insulator (dielectric substance) which transmits excitation light with which the quantum sensor is irradiated and fluorescence which is emitted from the quantum sensor. The second substrate 22 is a substrate in which an opening 221 that functions as a chamber is formed, and is laminated on the first substrate 21. The second substrate 22 may be made of an arbitrary material. Examples of the material include an insulator (dielectric substance) having low heat conductivity. The third substrate 23 functions as a lid of the chamber, and is laminated on the second substrate 22 after a liquid 26 (in the present embodiment, pure water) containing a cell is injected into the opening 221. The third substrate 23 may be made of an arbitrary material. Examples of the material include an insulator (dielectric substance) having low heat conductivity. In the present embodiment, quartz glass is used as the material of the first substrate 21, the second substrate 22, and the third substrate 23.

The cell retention device 2 further includes the intracellular quantum measurement device 1 (the substrate 10 on which the nanopillars 11 are formed), a first electrode 24a, and a second electrode 24b. The intracellular quantum measurement device 1 is laminated on the first substrate 21 inside the opening 221. Here, the intracellular quantum measurement device 1 is laminated on the first substrate 21 such that the back surface of the substrate 10 on which the nanopillars 11 are not formed is in contact with the surface of the first substrate 21. The intracellular quantum measurement device 1 may be fixed to a frame in order to facilitate handling. In this case, the intracellular quantum measurement device 1 is fixed to the first substrate 21 via the frame.

The first electrode 24a and the second electrode 24b are each a strip-shaped conductor and are laminated on the first substrate 21. The first electrode 24a is laminated on the first substrate 21 such that an outer edge thereof extends along a first side of the intracellular quantum measurement device 1. The second electrode 24b is laminated on the first substrate 21 such that an outer edge thereof extends along a second side (opposite side of the first side) of the intracellular quantum measurement device 1. The first electrode 24a and the second electrode 24b are short-circuited by a conducting wire 24c which diagonally crosses the intracellular quantum measurement device 1. The conducting wire 24c functions as an antenna which radiates an electromagnetic wave to be applied to the quantum sensor when a high-frequency current is inputted to the first electrode 24a and the second electrode 24b.

An opening 222a and an opening 232a are formed in the second substrate 22 and the third substrate 23, respectively, so that the first electrode 24a can be accessed from the upper surface side of the cell retention device 2. Similarly, an opening 222b and an opening 232b are formed in the second substrate 22 and the third substrate 23, respectively, so that the second electrode 24b can be accessed from the upper surface side of the cell retention device 2. Thus, it is possible to easily connect a high-frequency current source to the first electrode 24a and the second electrode 24b.

It is preferable that a width W of each of the first electrode 24a and the second electrode 24b is 0.1 mm or more and 500 mm or less. This makes it possible to apply an electromagnetic field (electromagnetic wave) in a wide frequency band from several megahertz to several gigahertz to a cell in the liquid 26. In the present embodiment, it is assumed that an electromagnetic field (electromagnetic wave) from 1 MHz to 120 GHz is applied, and the width W of each of the first electrode 24a and the second electrode 24b is set to 2.6 mm.

A total T+U of a thickness U of the first substrate 21 of the cell retention device 2 and a thickness T of the substrate 10 of the intracellular quantum measurement device 1 is preferably 0.3 mm or less. Thus, irradiation with excitation light and detection of fluorescence can be achieved using an objective lens which is widely used in fluorescence observation of cells. In the present embodiment, it is assumed that an objective lens having a working distance Wd of 0.3 mm is used. The thickness U of the first substrate 21 of the cell retention device 2 and the thickness T of the substrate 10 of the intracellular quantum measurement device 1 are set so that the total T+U is 0.21 mm.

By using the cell retention device 2 in accordance with the present embodiment, it is possible to insert the nanopillar 11 of the intracellular quantum measurement device 1 into a cell which is cultured in the liquid 26 and easily measure a state inside the cell. Moreover, by inputting a high-frequency current from the high-frequency current source to the first electrode 24a and the second electrode 24b, it is possible to easily measure a state inside a cell in an electromagnetic field (electromagnetic wave).

Note that the first electrode 24a and the second electrode 24b may not be short-circuited. In this case, when a voltage is applied between the first electrode 24a and 24b, an electric field corresponding to the voltage can be applied to the quantum sensor.

### [Intracellular quantum measurement system]

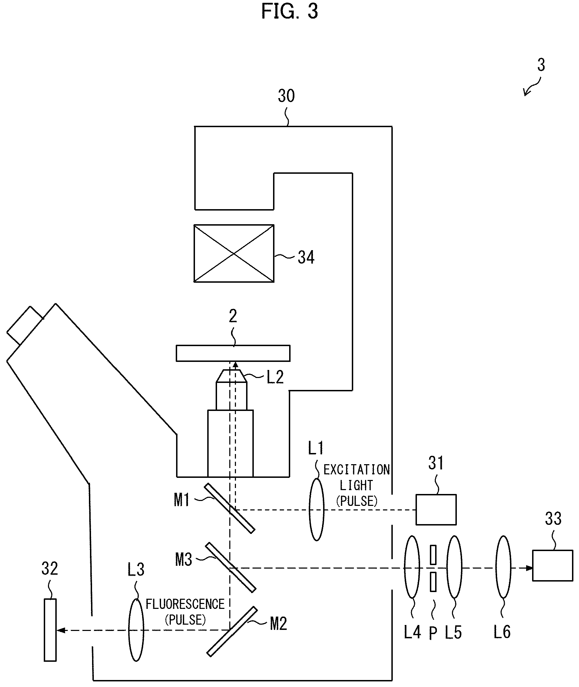

The following description will discuss an intracellular quantum measurement system 3 including the foregoing cell retention device 2, with reference to Fig. 3. Fig. 3 is a configuration diagram illustrating the intracellular quantum measurement system 3.

The intracellular quantum measurement system 3 includes the cell retention device 2, a housing 30, a light emission section 31, a light reception section 32, a single photon counter 33, first through sixth lenses L1 through L6, first through third mirrors M1 through M3, and a pinhole P. The intracellular quantum measurement system 3 has a wide field microscope mode and a confocal microscope mode. In order to switch between these modes, the first lens L1 and the third mirror M3 are movable.

The housing 30 is configured to accommodate the first through third lenses L1 through L3 and the first through third mirrors M1 through M3. In the present embodiment, a housing of an existing inverted microscope is used as the housing 30.

The light emission section 31 is configured to emit excitation light with which the quantum sensor is to be irradiated. In the present embodiment, an LED that emits laser light with a wavelength of 532 nm is used as the light emission section 31.

Meanwhile, in the wide field microscope mode, the first lens L1 is disposed in the optical path of excitation light, and the third mirror M3 is removed from the optical path of fluorescence. Therefore, the excitation light (collimated light) from the light emission section 31 is transformed into convergent light by the first lens L1, and then reflected by the first mirror M1 and enters the second lens L2. The second lens L2 transforms excitation light which is convergent light into collimated light, and the intracellular quantum measurement device 1 (more precisely, the surface of the substrate 10) in the cell retention device 2 is irradiated with the collimated light in a planar form. Fluorescence (divergent light) from the intracellular quantum measurement device 1 is collimated by the second lens L2 and then reflected by the second mirror M2. The fluorescence reflected by the second mirror M2 is condensed by the third lens L3 and enters the light reception section 32.

In the confocal microscope mode, the first lens L1 is removed from the optical path of excitation light, and the third mirror M3 is disposed in the optical path of fluorescence. Therefore, the excitation light (collimated light) from the light emission section 31 is reflected by the first mirror M1 and enters the second lens L2. The second lens L2 transforms excitation light which is collimated light into convergent light, and the intracellular quantum measurement device 1 (more precisely, the surface of the substrate 10) in the cell retention device 2 is irradiated with the convergent light in a spot form. Fluorescence (divergent light) from the intracellular quantum measurement device 1 is collimated by the second lens L2 and then reflected by the third mirror M3. The fluorescence reflected by the third mirror M3 is transformed into convergent light by the fourth lens L4, passes through the pinhole P, and is then collimated by the fifth lens L5. Then, the light is condensed by the sixth lens L6, and enters the single photon counter 33.

Note that the first mirror M1 needs to reflect excitation light and to allow fluorescence to pass therethrough. Therefore, in the present embodiment, a dichroic mirror is used as the first mirror M1. The first mirror M1 receives, together with fluorescence from the intracellular quantum measurement device 1, excitation light which has been reflected by the cell retention device 2. However, the excitation light is reflected by the first mirror M1, and is therefore not guided to the light reception section 32.

The light reception section 32 is configured, in the wide field microscope mode, to receive fluorescence emitted from the quantum sensor. In the present embodiment, a high-sensitivity imaging camera, specifically, an electron multiplying charge coupled device (EMCCD) is used as the light reception section 32. The single photon sensor 33 is configured, in the confocal microscope mode, to receive fluorescence emitted from the quantum sensor. In the present embodiment, 34 avalanche photodiode (APD) is used as the single photon sensor 33.

Note that, in a case where an intracellular state is measured using the intracellular quantum measurement system 3, in each measurement cycle, the light emission section 31 outputs one pulse of excitation light, and the light reception section 32 detects one pulse of fluorescence outputted, as a response thereto, from the quantum sensor. A time taken to carry out each measurement cycle is approximately 5 psec. Meanwhile, a frame rate of a video outputted from a high-sensitivity image sensor is approximately 50 frames per second. That is, a time (hereinafter, also referred to as a "read period") taken to read one image from the high image sensor is approximately 20 msec which is approximately 4000 times the time taken to carry out each measurement cycle. Therefore, the intracellular quantum measurement system 3 employs a configuration in which, in each read period, the measurement cycle is carried out N times (N is a natural number of 2 or more; in the present embodiment, N is approximately 1000) so as to multiply, by N, effective sensitivity of the high-sensitivity image sensor.

According to the intracellular quantum measurement system 3 in accordance with the present embodiment, it is possible to achieve both wide field measurement in the wide field microscope mode and high-resolution and high-sensitivity measurement in the confocal microscope mode. Moreover, according to the intracellular quantum measurement system 3 in accordance with the present embodiment, it is possible to easily switch between the wide field microscope mode and the confocal microscope mode without making contact with a subject to be measured.

The intracellular quantum measurement system 3 may further include a magnetic field fixation device 34. In quantum measurement, it is necessary to adjust a direction of a magnetic field acting on a quantum sensor with accuracy of 10 degrees or less. Under the circumstances, by using the magnetic field fixation device 34, it is possible to accurately adjust the direction of the magnetic field acting on the quantum sensor with respect to four directions (directions of three axes + rotation direction). Such adjustment is achieved, for example, by carrying out optically detected magnetic resonance (ODMR) measurement while changing the direction of the magnetic field using the magnetic field fixation device 34 and determining the direction of the magnetic field by referring to contrast and fluorescence intensity of the measurement result. Thus, for example, it is possible to accurately carry out high magnetic field application which is important in order to achieve high-frequency resolution in structural analysis such as NMR and ESR.

### (Additional remarks regarding effects)

According to the intracellular quantum measurement system 3, it is possible to carry out optimized fluorescence extraction in a state in which a quantum measurement material is introduced into a biometric sample in various forms. Further, by using a general-purpose computer (not illustrated) in combination, it is possible to carry out optically detected magnetic resonance (ODMR) measurement while precisely controlling an environment such as a direction of a magnetic field, temperature, and humidity. Here, ODMR measurement is a measurement method of measuring a quantum state change of a quantum sensor by carrying out irradiation with excitation light and intensity measurement of fluorescence while changing a frequency of a microwave. In this case, the intracellular quantum measurement device 1 is used to introduce the quantum sensor into a cell, the cell retention device 2 is used to apply a microwave to the quantum sensor which is introduced into the cell, and the intracellular quantum measurement system 3 is used to detect intensity of fluorescence emitted from the quantum sensor which is introduced into the cell. In ODMR measurement, a computer (not illustrated) is used to control timing of irradiation with excitation light and timing of application of a microwave, and is also used to calculate a quantum state of the quantum sensor from the detected intensity of fluorescence.

Further, by carrying out application of a microwave and intensity detection of fluorescence with a time resolution of 400 psec or less, it is possible to achieve advanced quantum manipulation such as rabi and echo. Further, by increasing the number of microwaves to be applied in micro region spin measurement and controlling spin other than the quantum sensor, it is possible to achieve double electron-electron resonance (DEER).

### Examples

The inventors carried out intracellular quantum measurement of an Hepa 1-6 cell using the intracellular quantum measurement system 3. In the intracellular quantum measurement device 1 used, nanopillars 11 each having a height of 3.2 µm and a diameter of 300 nm were formed at regular intervals of 15 µm on a surface of a substrate having a thickness of 40 µm.

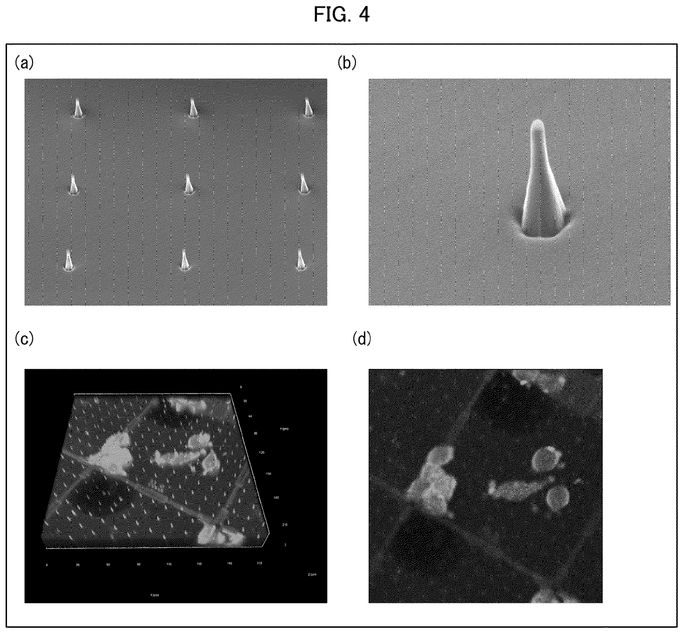

(a) of Fig. 4 shows an image obtained by imaging a surface of the intracellular quantum measurement device 1. (b) of Fig. 4 shows an image obtained by imaging the nanopillar 11 of the intracellular quantum measurement device 1. (c) and (d) of Fig. 4 each show an image obtained by imaging a Hepa 1-6 cell which is cultured in the cell retention device 2. Note that the Hepa 1-6 cell was stained in advance using a staining reagent for cell membrane. As the staining reagent for cell membrane, CellMask (registered trademark) Green was used. In (c) and (d) of Fig. 4, a state is shown in which the nanopillar 11 is inserted into the Hepa 1-6 cell.

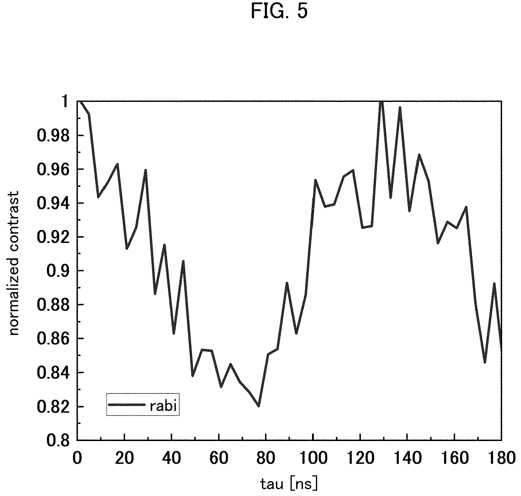

The inventors carried out Rabi measurement in a state in which the nanopillar 11 was inserted into a cell using the intracellular quantum measurement system 3. Fig. 5 is a graph illustrating τ dependency of normalized contrast obtained by the Rabi measurement. Here, the normalized contrast is a dimensionless quantity which represents a state transition (i.e., |0> and |±1> where an NV center is used as a qubit) of electron spin by microwave manipulation. Further, τ is an amount representing a microwave application time, and a unit thereof is nanoseconds. From the graph illustrated in Fig. 5, it can be seen that a state transition of electron spin corresponding to the microwave application time was achieved. Thus, it was confirmed that quantum manipulation with high contrast in the cell was possible.

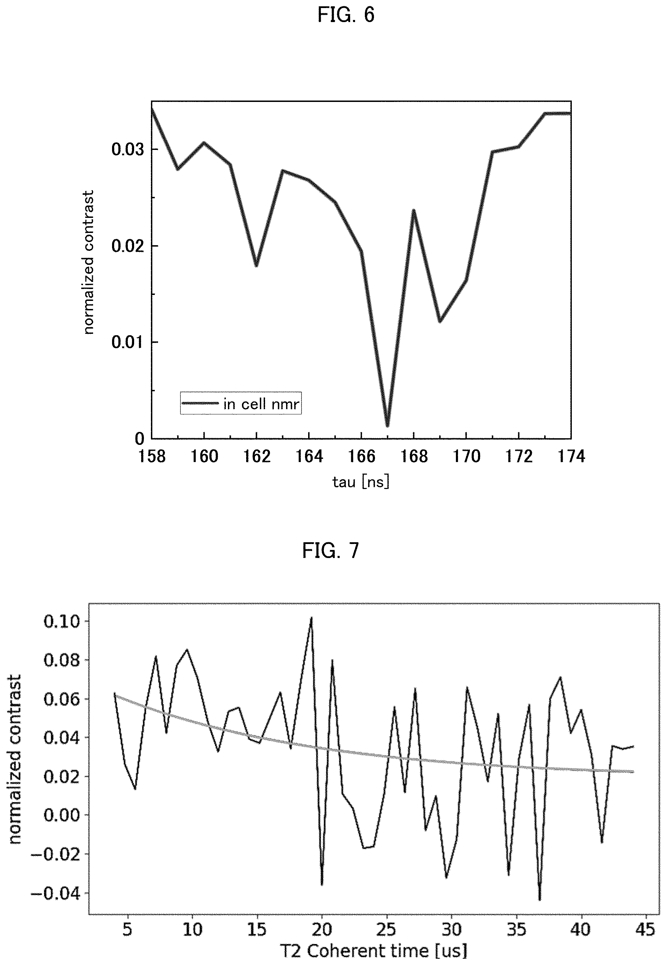

Moreover, the inventors carried out NMR measurement in a state in which the nanopillar 11 was inserted into a cell using the intracellular quantum measurement system 3. Fig. 6 is a graph illustrating τ dependency of normalized contrast obtained by the NMR measurement. From the graph illustrated in Fig. 6, it can be seen that a peak (dip) corresponding to nuclear spin which exists in a cell appears in a region in which τ is 164 nsec or more and 171 nsec or less. Even in a case where NMR measurement was carried out in a state in which the nanopillar 11 was not inserted into a cell, such a peak (dip) did not appear. Thus, it was confirmed that quantum measurement of an intracellular state can be achieved using the nanopillar 11.

Further, the inventors measured, using the intracellular quantum measurement system 3, time dependency of normalized contrast in a state in which the nanopillar 11 was inserted into a cell. Fig. 7 is a graph illustrating time dependency of normalized contrast obtained by the measurement. From the graph illustrated in Fig. 7, it can be seen that a coherence time (T2) is 15.9 psec. Thus, in the quantum sensor included in the tip of the nanopillar 11 inserted into the cell, it was confirmed that quantum property thereof was maintained for a sufficiently long time.

As described above, the inventors were able to detect nuclear spin in an intracellular micro region by using the nanopillar 11. In addition to the quantum manipulation of the quantum sensor, the inventors carried out quantum manipulation of electron spin in the vicinity of the quantum sensor. The following description will discuss details thereof.

The inventors, at the tip part 11a of the nanopillar 11, manipulated (double quantum manipulation) (i) a quantum state of the quantum sensor (NV center) inserted into a cell and (ii) electron spin (quantum state) of a molecule, an atom, an ion, or a radical (hereinafter, referred to as "radical or the like") in the cell that existed around the tip part 11a (quantum sensor) of the nanopillar 11 that was inserted into the cell.

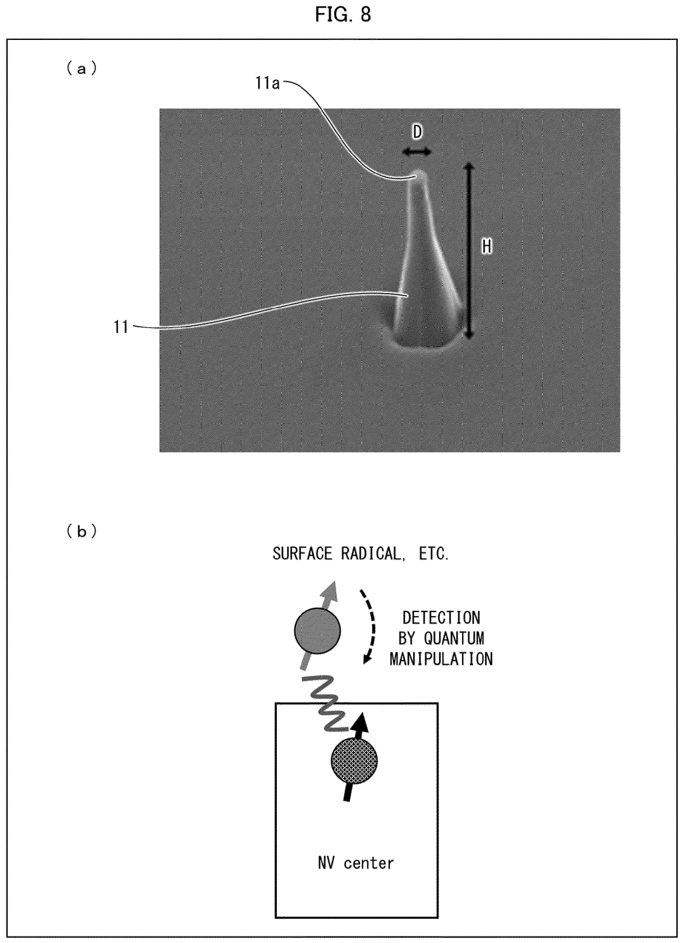

(a) of Fig. 8 is a diagram illustrating the nanopillar 11, and (b) of Fig. 8 is an expanded schematic diagram illustrating a quantum sensor (NV center) which is disposed at the tip part 11a of the nanopillar 11. In this Example, the height H of the nanopillar 11 was 3.2 µm, and the diameter D of the tip part 11a of the nanopillar 11 was 300 nm. The NV center was disposed at a depth of approximately 10 nm from the surface of the tip part 11a of the nanopillar 11, and was capable of detecting quantum manipulation of a surface radical in the vicinity of the tip part 11a (quantum sensor). Here, the foregoing area around the tip part 11a includes areas around (i) a bulged curvature surface which constitutes the leading edge surface of the tip part 11a and (ii) an outer peripheral surface which constitutes a columnar side surface of the tip part 11a.

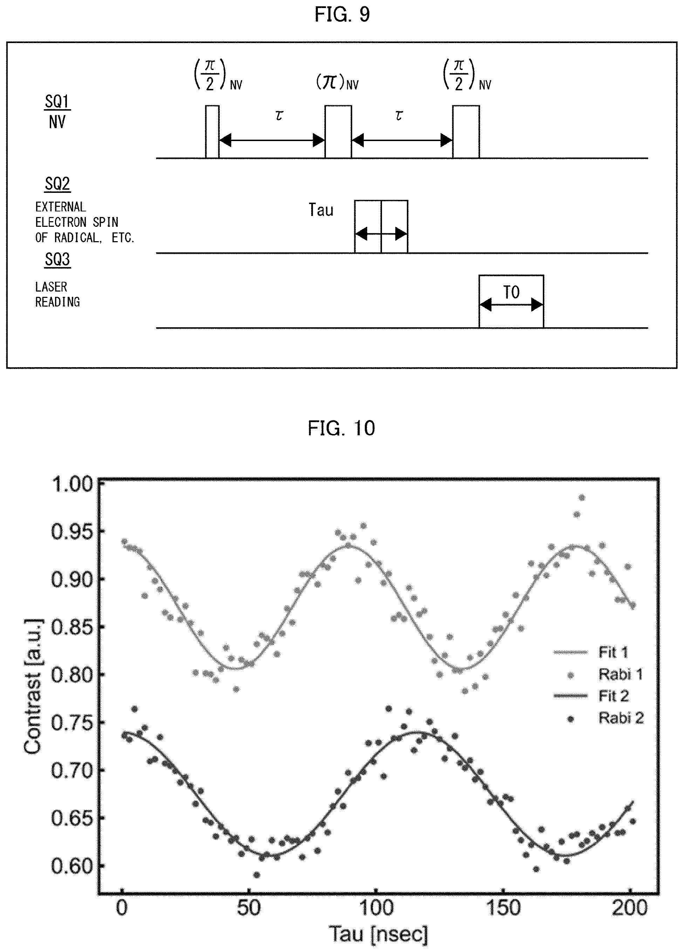

Fig. 9 is a diagram illustrating a sequence for carrying out quantum manipulation on a quantum sensor and a radical or the like in the vicinity of the quantum sensor in the nanopillar 11 illustrated in Fig. 8. A sequence SQ1 represents a pulse sequence in which a microwave is applied to the quantum sensor (NV center). A sequence SQ2 represents a pulse sequence in which a microwave is applied to (electron spin of) a radical or the like. A sequence SQ3 represents a pulse sequence for laser reading.

These sequences SQ1 through SQ3 can be considered to correspond to the following processes (1) through (3), respectively. That is, the intracellular quantum measurement method can be constituted by the processes (1) through (3).
(1) A first quantum manipulation process of applying pulses of first electromagnetic waves (e.g., microwaves) to the quantum sensor (NV center) to manipulate a quantum state of the quantum sensor.
(2) A second quantum manipulation process of applying pulses of second electromagnetic waves (e.g., microwaves), which have a frequency different from that of the first electromagnetic waves, to a radical or the like (at least any of a molecule, an atom, an ion, and a radical) in a cell that exists around the tip part 11a so as to manipulate a quantum state (electron state) of the radical or the like.
(3) A state detection process of irradiating the quantum sensor and a radical or the like with excitation light (e.g., pulses of laser light) and receiving fluorescence from the quantum sensor and the radical or the like.

In the sequence SQ1, pulses of microwaves are applied to the NV center at time intervals τ to switch states (π/2, π) of the NV center. In the sequence SQ2, pulses of microwaves having a spin manipulation time (pulse width) Tau are applied to external electron spin of the radical or the like. These microwaves (electromagnetic waves) are applied from the conducting wire 24c (antenna) as described above.

In the sequence SQ3, for example, irradiation with laser light (excitation light) having a pulse width of 300 nsec to 500 nsec is carried out, and fluorescence from the quantum sensor and the radical or the like is received. As described above, the excitation light is emitted from the light emission section 31, and the fluorescence is received by the single photon counter 33 or the light reception section 32.

The sequences SQ1 through SQ3 can be controlled independently of each other. By controlling timings of the sequences SQ1 through SQ3, it is possible to confirm manipulation of electron spin of the radical or the like in the vicinity of the quantum sensor.

Fig. 10 is a graph illustrating a working example of a result obtained using the pulse sequence illustrated in Fig. 9, and represents tau dependency of contrast obtained by ESR measurement using double quantum manipulation. Graphs "Fit 1" and "Fit 2" are different in intensity of a microwave applied to a radical. The graph "Fit 1" has greater intensity of a microwave applied to the radical than the graph "Fit 2". In both the graphs "Fit 1" and "Fit 2", rotational motion of a radical is measured by changing the spin manipulation time Tau. By decreasing the intensity of the applied microwave from the graph "Fit 1" to the graph "Fit 2", it can be seen that an oscillation period called Rabi oscillation is reduced. That is, with respect to microwaves, an ESR reaction is detected and it can be confirmed that quantum manipulation is carried out.

Measurement and quantum manipulation of electron spin in a micro region (e.g., a volume of approximately (5 nm)³) have been utilized, to date, for detection of a single protein formed on a thin film and structural analysis thereof, and detection of a single DNA and structural analysis thereof. However, such measurement, quantum manipulation, and the like have not been carried out in cells. The inventors have enabled detection and quantum manipulation of a surface radical by forming a single NV center at the tip of a diamond pillar.

The quantum measurement method by the inventors in which quantum manipulation is carried out on both a quantum sensor and a radical or the like makes it possible to carry out various intracellular measurements. Examples of such intracellular measurements include: measurement of a single DNA; structural analysis of a cryptochrome protein which is considered to be associated with directional detection of a migratory bird; identification and detection of ions; and structural analysis of various radicals generated in intracellular oxidation phenomena.

Aspects of the present invention can also be expressed as follows:
An intracellular quantum measurement device in accordance with aspect 1 includes: a substrate that has a surface on which a nanopillar is formed, the nanopillar being to be inserted into a cell, and the nanopillar having a tip part in which a quantum sensor is included.

According to the above configuration, it is possible to achieve quantum measurement that can measure an intracellular state and that overcomes disadvantages of quantum measurement that uses nanoparticles.

The intracellular quantum measurement device in accordance with aspect 2 employs, in addition to the feature of the intracellular quantum measurement device in accordance with aspect 1, a feature in which a height of the nanopillar is 1 µm or more and 20 µm or less.

According to the above configuration, it is possible to achieve intracellular quantum measurement while satisfying both a physical condition which is to sufficiently enhance fluorescence extraction efficiency and a biological condition which is to sufficiently reduce toxicity with respect to cells.

The intracellular quantum measurement device in accordance with aspect 3 employs, in addition to the feature of the intracellular quantum measurement device in accordance with aspect 1 or 2, a feature in which a diameter of the tip part of the nanopillar is 100 nm or more and 1000 µm or less.

According to the above configuration, it is possible to achieve intracellular quantum measurement while satisfying both a physical condition which is to sufficiently enhance fluorescence extraction efficiency and a biological condition which is to sufficiently reduce toxicity with respect to cells.

The intracellular quantum measurement device in accordance with aspect 4 employs, in addition to the feature of the intracellular quantum measurement device in accordance with any one of aspects 1 through 3, a feature in which a plurality of nanopillars which are to be respectively inserted into a plurality of cells are formed on the surface of the substrate, the plurality of nanopillars each having a tip part in which a quantum sensor is included.

According to the above configuration, it is possible to simultaneously measure a plurality of intracellular states.

The intracellular quantum measurement device in accordance with aspect 5 employs, in addition to the feature of the intracellular quantum measurement device in accordance with aspect 4, a feature in which an interval between adjacent nanopillars in the plurality of nanopillars is 10 µm or more and 60 µm or less.

According to the above configuration, it is possible to insert the nanopillars into cells more reliably. As a result, it is possible to carry out quantum measurement of intracellular states more reliably.

The intracellular quantum measurement device in accordance with aspect 6 employs, in addition to the feature of the quantum measurement device in accordance with any one of aspects 1 through 5, a feature in which the nanopillar and the substrate are constituted by diamond crystal; and the quantum sensor is an NV center.

According to the above configuration, it is possible to carry out quantum measurement of an intracellular state with high accuracy.

The intracellular quantum measurement device in accordance with aspect 7 employs, in addition to the feature of the intracellular quantum measurement device in accordance with aspect 6, a feature in which a <111> direction of the diamond crystal conforms to a normal direction to the surface of the substrate.

According to the above configuration, it is possible to control, with high accuracy, a magnetic field which is applied to an intracellular quantum sensor.

A cell retention device in accordance with aspect 8 includes: a first substrate; a second substrate which is laminated on the first substrate and in which an opening is formed, the opening functioning as a chamber for storing a liquid containing a cell; the intracellular quantum measurement device described in any one of aspects 1 through 7 which is laminated on the first substrate in the opening; and an electrode which is laminated on the intracellular quantum measurement device in the opening.

According to the above configuration, it is possible to easily carry out quantum measurement of a state of a cell which is cultured in a liquid stored in the chamber.

In an intracellular quantum measurement method in accordance with aspect 9, a substrate that has a surface on which a nanopillar is formed is used to carry out quantum measurement in a state in which the nanopillar is inserted into a cell, the nanopillar having a tip part in which a quantum sensor is included.

According to the above configuration, it is possible to achieve quantum measurement that can measure an intracellular state and that overcomes disadvantages of quantum measurement that uses nanoparticles.

The intracellular quantum measurement method in accordance with aspect 10 employs, in addition to the feature of the intracellular quantum measurement method in accordance with aspect 9, a feature in which a substrate that has a surface on which a plurality of nanopillars are formed is used to carry out quantum measurement in a state in which the plurality of nanopillars are respectively inserted into a plurality of cells, the plurality of nanopillars each having a tip part in which a quantum sensor is included.

According to the above configuration, it is possible to simultaneously measure a plurality of intracellular states.

The intracellular quantum measurement method in accordance with aspect 11 includes, in addition to the feature of the intracellular quantum measurement method in accordance with aspect 9 or 10: a first quantum manipulation process of applying pulses of first electromagnetic waves to the quantum sensor so as to manipulate a quantum state of the quantum sensor; a second quantum manipulation process of applying pulses of second electromagnetic waves to at least any of a molecule, an atom, an ion, and a radical that exist around the tip part so as to manipulate a quantum state of at least any of the molecule, atom, ion, and radical, the second electromagnetic waves being different in frequency from the first electromagnetic waves; and a state detection process of irradiating the quantum sensor and at least any of the molecule, atom, ion, and radical with excitation light and receiving fluorescence from the quantum sensor and at least any of the molecule, atom, ion, and radical.

According to the above configuration, by carrying out quantum manipulation on both the quantum sensor and the radical or the like, it is possible to obtain various kinds of information.

### (Additional remarks)

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

1: Intracellular quantum measurement device
10: Substrate
11: Nanopillar
2: Cell retention device
3: Intracellular quantum measurement system

## Claims

1. An intracellular quantum measurement device, comprising:
a substrate that has a surface on which a nanopillar is formed, the nanopillar being to be inserted into a cell, and the nanopillar having a tip part in which a quantum sensor is included.

2. The intracellular quantum measurement device as set forth in claim 1, wherein:
a height of the nanopillar is 1 µm or more and 20 µm or less.

3. The intracellular quantum measurement device as set forth in claim 1 or 2, wherein:
a diameter of the tip part of the nanopillar is 100 nm or more and 1000 µm or less.

4. The intracellular quantum measurement device as set forth in any one of claims 1 through 3, wherein:
a plurality of nanopillars which are to be respectively inserted into a plurality of cells are formed on the surface of the substrate, the plurality of nanopillars each having a tip part in which a quantum sensor is included.

5. The intracellular quantum measurement device as set forth in claim 4, wherein:
an interval between adjacent nanopillars in the plurality of nanopillars is 10 µm or more and 60 µm or less.

6. The intracellular quantum measurement device as set forth in any one of claims 1 through 5, wherein:
the nanopillar and the substrate are constituted by diamond crystal; and
the quantum sensor is an NV center.

7. The intracellular quantum measurement device as set forth in claim 6, wherein:
a <111> direction of the diamond crystal conforms to a normal direction to the surface of the substrate.

8. A cell retention device, comprising:
a first substrate;
a second substrate which is laminated on the first substrate and in which an opening is formed, the opening functioning as a chamber for storing a liquid containing a cell;
an intracellular quantum measurement device recited in any one of claims 1 through 7 which is laminated on the first substrate in the opening; and
an electrode which is laminated on the intracellular quantum measurement device in the opening.

9. An intracellular quantum measurement method, wherein:
a substrate that has a surface on which a nanopillar is formed is used to carry out quantum measurement in a state in which the nanopillar is inserted into a cell, the nanopillar having a tip part in which a quantum sensor is included.

10. The intracellular quantum measurement method as set forth in claim 9, wherein:
a substrate that has a surface on which a plurality of nanopillars are formed is used to carry out quantum measurement in a state in which the plurality of nanopillars are respectively inserted into a plurality of cells, the plurality of nanopillars each having a tip part in which a quantum sensor is included.

11. The intracellular quantum measurement method as set forth in claim 9 or 10, comprising:
a first quantum manipulation process of applying pulses of first electromagnetic waves to the quantum sensor so as to manipulate a quantum state of the quantum sensor;
a second quantum manipulation process of applying pulses of second electromagnetic waves to at least any of a molecule, an atom, an ion, and a radical that exist around the tip part so as to manipulate a quantum state of at least any of the molecule, atom, ion, and radical, the second electromagnetic waves being different in frequency from the first electromagnetic waves; and
a state detection process of irradiating the quantum sensor and at least any of the molecule, atom, ion, and radical with excitation light and receiving fluorescence from the quantum sensor and at least any of the molecule, atom, ion, and radical.
